(19) Europäisches Patentamt
European Patent Office
Office européen des brevets

(11) **EP 4 379 349 A1**

(12) **EUROPEAN PATENT APPLICATION**
published in accordance with Art. 153(4) EPC

(43) Date of publication:
**05.06.2024 Bulletin 2024/23**

(21) Application number: **22855827.6**

(22) Date of filing: **03.08.2022**

(51) International Patent Classification (IPC):
**G01N 5/02** *(2006.01)*    **G01N 27/00** *(2006.01)*

(52) Cooperative Patent Classification (CPC):
**G01N 5/02; G01N 27/00**

(86) International application number:
**PCT/JP2022/029810**

(87) International publication number:
**WO 2023/017764 (16.02.2023 Gazette 2023/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.08.2021 JP 2021131733**

(71) Applicant: **I-PEX Inc.**
**Fushimi-ku, Kyoto-shi**
**Kyoto 612-8024 (JP)**

(72) Inventors:
• **OGATA Kenji**
**Ogori-shi, Fukuoka 838-0106 (JP)**
• **KUROGI Shogo**
**Ogori-shi, Fukuoka 838-0106 (JP)**

(74) Representative: **SSM Sandmair**
**Patentanwälte Rechtsanwalt**
**Partnerschaft mbB**
**Joseph-Wild-Straße 20**
**81829 München (DE)**

(54) **SUBSTANCE DETECTION SYSTEM**

(57)    A substance detection system (1A) includes a substance sensor (10), a distance sensor (11), and an information processor (20). The substance sensor (10) detects a substance (3) emitted from a generation source (2). The distance sensor (11) detects distance (L) between the generation source (2) and the substance sensor (10). The information processor (20) includes a determiner (21). The determiner (21) determines whether or not the distance (L) detected by the distance sensor (11) falls within a range from (L1) to (L2) where a detection result by the substance sensor (10) is determined to be valid.

FIG.1

**Description**

Technical Field

The present disclosure relates to a substance detection system.

Background Art

In Patent Literature 1, a chemical sensor device that detects a substance emitted from a generation source, based on the amount of change in a resonant frequency of an oscillator that occurs when the substance is adsorbed or detached is disclosed.

Citation List

Patent Literature

Patent Literature 1: Unexamined Japanese Patent Application Publication No. 2009-204584

Summary of Invention

Technical Problem

In the above-described chemical sensor device, there is an inconvenience that a detection result of a substance varies depending on distance between a generation source of the substance and the chemical sensor device. Suppressing the variation leads to stabilization of precision of a detection result.
The present disclosure has been made in consideration of the above-described circumstances, and an objective of the present disclosure is to provide a substance detection system capable of stabilizing precision of a detection result.

Solution to Problem

In order to achieve the above-described objective, a substance detection system according to a first aspect of the present disclosure includes:

a substance sensor to detect a substance emitted from a detection target;
a distance sensor to detect a distance between the detection target and the substance sensor; and
a determiner to determine whether or not distance detected by the distance sensor falls within a range where a detection result by the substance sensor is determined to be valid.

In this case, the substance detection system may include a notifier to notify that a substance can be detected by the substance sensor when the determiner determines that distance detected by the distance sensor falls within the range.
The notifier of the substance detection system may notify that a substance cannot be detected by the substance sensor when the determiner determines that distance detected by the distance sensor falls outside the range.
The substance detection system may include a controller to start detection of a substance by the substance sensor when the determiner determines that distance detected by the distance sensor falls within the range.
The controller of the substance detection system may suspend detection of a substance by the substance sensor when the determiner determines that distance detected by the distance sensor falls outside the range.
The substance detection system may include a range storage to store the range with respect to each of the detection targets, wherein
the determiner may perform determination by reading the range corresponding to the detection target from the range storage.
The substance detection system may include a detection result storage to store a detection result by the substance sensor and a detection result by the distance sensor in association with each other.
A substance detection system according to a second aspect of the present disclosure includes:

a substance sensor to detect a substance emitted from a detection target;
a distance sensor to detect a distance between the detection target and the substance sensor; and
a first corrector to correct a detection result by the substance sensor, based on a detection result by the distance sensor.

In both the first aspect and the second aspect, the substance detection system may include:

an environment sensor to detect information about an ambient environment; and
a second corrector to correct a detection result by the substance sensor, based on a detection result by the environment sensor.

The substance sensor and the distance sensor of the substance detection system may be integrated with each other.

The substance detection system may include

a main body; and
an adapter attachable and detachable to and from the main body, wherein
the substance sensor and the distance sensor may be installed in the adapter.

The substance sensor and the distance sensor of the substance detection system may be separable from each other.

The substance sensor, the distance sensor, and the environment sensor of the substance detection system may be integrated with one another.

The substance detection system may include

a main body; and
an adapter attachable and detachable to and from the main body, wherein
the substance sensor, the distance sensor, and the environment sensor may be installed in the adapter.

At least two of the substance sensor, the distance sensor, or the environment sensor of the substance detection system may be separable from each other.

Advantageous Effects of Invention

According to the present disclosure, the substance detection system includes the determiner to determine whether or not the distance between a detection target and the substance sensor falls within a range where a detection result by the substance sensor is determined to be valid. Thus, whether or not a detection result by the substance sensor is valid can be determined. As a result, precision of a detection result of the substance sensor can be stabilized.

Brief Description of Drawings

FIG. 1 is a block diagram illustrating a configuration of a substance detection system according to Embodiment 1 of the present disclosure;
FIG. 2 is a block diagram illustrating a hardware configuration of the substance detection system in FIG. 1;
FIG. 3 is a flowchart illustrating substance detection processing performed by an information processor constituting the substance detection system in FIG. 1;
FIG. 4 is a graph illustrating a relationship between distance from a generation source and a detection level by a substance sensor;
FIG. 5 is a block diagram illustrating a configuration of a substance detection system according to Embodiment 2 of the present disclosure;
FIG. 6 is a flowchart illustrating substance detection processing performed by an information processor constituting the substance detection system in FIG. 5;
FIG. 7 is a block diagram illustrating a configuration of a substance detection system according to Embodiment 3 of the present disclosure;
FIG. 8 is a schematic diagram illustrating an example of data stored in a range storage in FIG. 7;
FIG. 9 is a block diagram illustrating a configuration of a substance detection system according to Embodiment 4 of the present disclosure;
FIG. 10 is a schematic diagram illustrating an example of data stored in a detection result storage in FIG. 9;
FIG. 11 is a block diagram illustrating a configuration of a substance detection system according to Embodiment 5 of the present disclosure;

FIG. 12 is a graph illustrating a relationship between distance and a correction value;

FIG. 13 is a block diagram illustrating another example of the substance detection system in FIG. 11;

FIG. 14 is a block diagram illustrating a configuration of a substance detection system according to Embodiment 6 of the present disclosure;

FIG. 15 is a perspective view of a substance detection system according to Embodiment 7 of the present disclosure;

FIG. 16 is a perspective view of a substance detection system according to Embodiment 8 of the present disclosure;

FIG. 17A is a perspective view of a substance detection system according to Embodiment 9 of the present disclosure;

FIG. 17B is a perspective view illustrating a portion of the substance detection system in FIG. 17A;

FIG. 17C is a perspective view of an adapter constituting the substance detection system in FIG. 17A viewed from a different direction; and

FIG. 17D is a perspective view of the adapter constituting the substance detection system in FIG. 17A viewed from a direction different from the direction in FIG. 17C.

Description of Embodiments

Embodiments of the present disclosure are described in detail below with reference to the drawings. The same or equivalent constituent components are designated by the same reference numerals in the drawings.

Embodiment 1

First, Embodiment 1 of the present disclosure is described. A substance detection system according to the present embodiment detects a substance contained in a gas.

[Overall Configuration]

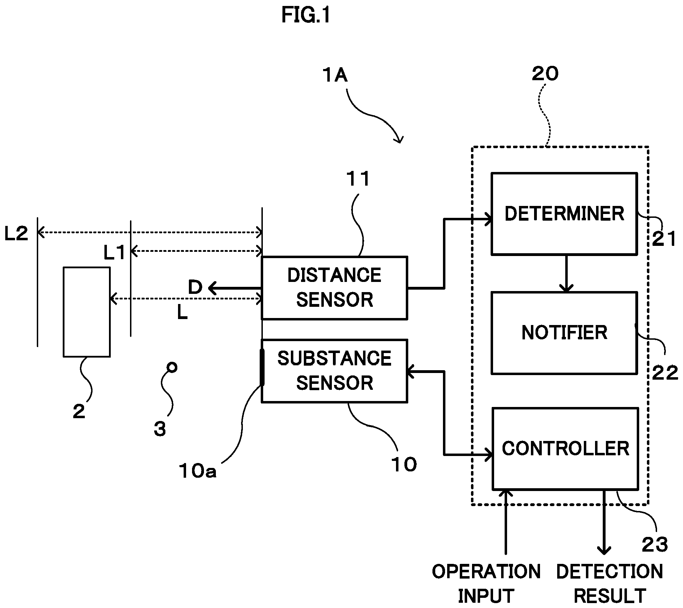

As illustrated in FIG. 1, a substance detection system 1A according to the present embodiment detects a substance 3 that is a detection target and is emitted from a generation source 2. The substance detection system 1A includes a substance sensor 10, a distance sensor 11, and an information processor 20.

The substance sensor 10 detects the substance 3 emitted from the generation source 2. The substance 3 emitted from the generation source 2 floats in the air. The substance sensor 10 includes an intake port 10a to take in the air. The substance sensor 10 takes in the substance 3 floating in the air from the intake port 10a. Note that although a pump to take in the air may be disposed to the substance sensor 10, a pump does not have to be disposed. The substance sensor 10 may be a substance sensor that only takes in the substance 3 from the intake port 10a.

A not-illustrated adsorption film that adsorbs the substance 3 is disposed inside the substance sensor 10, and taken-in air is adsorbed on the adsorption film. The substance sensor 10 detects a change in weight of the adsorption film on which the substance 3 is adsorbed. The change in weight can be calculated from, for example, a change in vibration frequency of the adsorption film. For example, the adsorption film is mounted on a beam that can be vibrated by a piezoelectric element, and the substance 3 can be detected based on a change in vibration amplitude of the beam. The larger the number of particles of the substance 3 adsorbed on the adsorption film is, the larger a detection level of the substance 3 by the substance sensor 10 becomes.

Note that the type of substance 3 to be detected is not limited to one type. A plurality of types of substances 3 is emitted from the generation source 2. The substance sensor 10 can be configured to include a sensitive film for detecting each of the plurality of types of substances 3 and to detect the plurality of types of substances 3.

The distance sensor 11 detects distance L to an object that exists in a specific direction D. When the generation source 2 is subjected to the detection target, a user of the substance detection system 1A points the distance sensor 11 in such a manner that the generation source 2 exists in the specific direction D. As the distance sensor 11, for example, an infrared proximity sensor is used. The substance sensor 10 and the distance sensor 11 are arranged close to each other, and the intake port 10a of the substance sensor 10 points in the specific direction D. Thus, distance to an object that is detected by the distance sensor 11 can be considered as distance between the substance sensor 10 and the object. The distance sensor 11 detects distance L between the generation source 2 and the substance sensor 10. Note that the distance sensor 11 is not limited to an infrared proximity sensor, and a detection principle of the distance sensor 11 is not limited to a specific detection principle as long as the detection principle enables the distance L to the generation source 2 to be measured.

The information processor 20 is an information processing device in which information processing executed by software is specifically achieved using hardware resources. The information processor 20 includes a determiner 21, a notifier 22, and a controller 23.

The determiner 21 determines whether or not the distance L detected by the distance sensor 11 falls within a range where a detection result by the substance sensor 10 is determined to be valid. In the present embodiment, the range where a detection result is determined to be valid is assumed to be a range of a distance L1 or more and a distance L2 or less. Hereinafter, the range is referred to as a range from L1 to L2.

The notifier 22 notifies that the substance 3 can be detected by the substance sensor 10 when the determiner 21 determines that the distance L detected by the distance sensor 11 falls within the range from L1 to L2. The notification is referred to as a detection enabled notification. Further, the notifier 22 notifies that the substance 3 cannot be detected by the substance sensor 10 when the determiner 21 determines that the distance L detected by the distance sensor 11 falls outside the range from L1 to L2. The notification is referred to as detection disabled notification.

The controller 23 accepts an operation input from the user. When instructed to detect the substance 3 by an operation input, the controller 23 controls the substance sensor 10 to detect the substance 3. A detection result of the substance 3 includes a detection amount of the substance 3.

[Hardware Configuration]

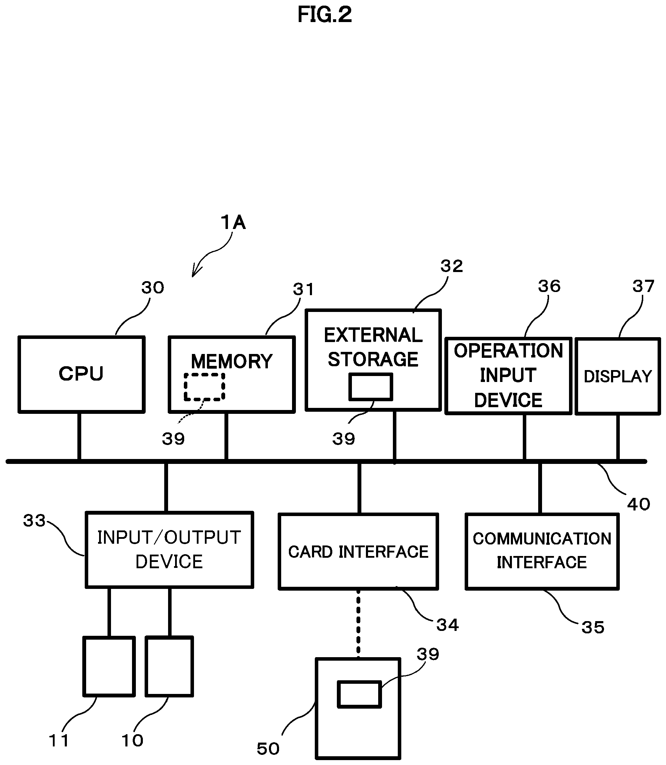

In FIG. 2, a hardware configuration of the substance detection system 1A is illustrated. As illustrated in FIG. 2, a portion of the substance detection system 1A related to the information processor 20 includes a central processing unit (CPU) 30, a memory 31, an external storage 32, an input/output device 33, a card interface 34, a communication interface 35, an operation input device 36, and a display 37. The respective constituent elements of the information processor 20 are connected to one another via an internal bus 40.

The CPU 30 is a processor (operation device) that executes a software program (hereinafter, simply referred to as "program"). Into the memory 31, a program 39 is read from the external storage 32, and the CPU 30 performs operation of the information processor 20 by executing the program 39 stored in the memory 31. The CPU 30 has a timer built-in and is capable of measuring time.

The memory 31 is, for example, a random access memory (RAM). In the memory 31, not only the program 39 that is executed by the CPU 30 but also data necessary for execution of the program 39 by the CPU 30 and data generated as a result of execution of the program 39 are stored.

The external storage 32 is, for example, a hard disk or the like. In the external storage 32, the program 39 that is to be executed by the CPU 30 is stored. In addition, in a portable non-transitory recording medium 50, such as a universal serial bus (USB) memory, the program 39 is stored. In the external storage 32, the program 39 that is transferred from the non-transitory recording medium 50 is stored. In addition, the external storage 32 stores the distances L1 and L2 in the range from L1 to L2, which is used for determination of distance.

The input/output device 33 is an interface through which data input and output to and from the substance sensor 10 and the distance sensor 11 are performed. Information about the substance 3 (an adsorption film the vibration frequency of which changes (the type of the substance 3) and a detection level) detected by the substance sensor 10 and the distance L detected by the distance sensor 11 are stored in the memory 31 via the input/output device 33.

The card interface 34 is an interface with the non-transitory recording medium 50. The program 39 is input via the card interface 34 and stored in the external storage 32.

The communication interface 35 is an interface for connection to a communication network, such as the Internet. The substance detection system 1A is connected to an external server computer or the like via the communication interface 35.

The operation input device 36 is a man-machine interface that is operated by the user. The operation input device 36 includes a pointing device such as a touch panel and a mouse, and a keyboard. An operation that is input to the operation input device 36 is sent to the CPU 30. The CPU 30 executes the program 39 in accordance with an instruction in the operation input. For example, an operation input to start detection is input to the operation input device 36.

The display 37 is a man-machine interface to display an image. The display 37 includes a cathode ray tube (CRT) or a liquid crystal display (LCD). On the display 37, a determination result of detected odor or the like is displayed. Note that the operation input device 36 and the display 37 may be combined as a touch panel. For example, the detection enabled notification and the detection disabled notification are displayed on the display 37. In addition, a detection result of the substance 3 is displayed on the display 37.

The operation input device 36 and the display 37 may be integrated with each other as a touch panel. In addition, in the substance detection system 1A, a voice input/output device to input and output a voice may be disposed. The voice input/output device is capable of inputting and outputting of a voice relating to an operation input to start detection, the detection enabled notification and the detection disabled notification, and a detection result of the substance 3. As described above, processing in the determiner 21, the notifier 22, and the controller 23 in FIG. 1 is achieved by the CPU 30 causing the substance sensor 10 and the distance sensor 11 to operate via the input/output device 33, operating in accordance with an operation input through the operation input device 36, and causing the display 37 or the like to operate.

[Substance Detection Processing]

Next, operation of the substance detection system 1A according to the present embodiment of the present disclosure is described. The following description is made mainly on substance detection processing that is performed by the information processor 20.

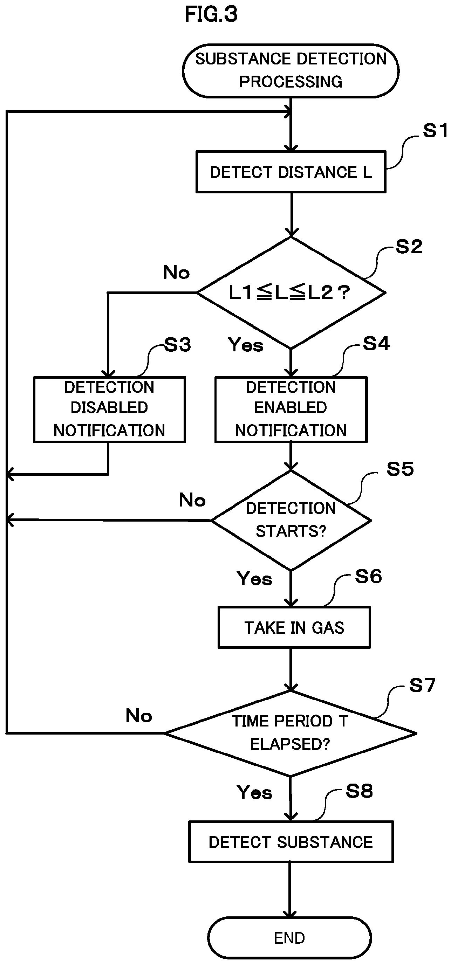

As illustrated in FIG. 3, first, the information processor 20 causes the distance sensor 11 to detect a distance L to the generation source 2 (step S1). Succeedingly, the determiner 21 of the information processor 20 determines whether or not the detected distance L falls within the range from L1 to L2, that is, $L1 \leq L \leq L2$ (step S2). When the distance L does not fall within the range from L1 to L2 (step S2; No), the notifier 22 of the information processor 20 performs detection disabled notification (step S3). After completion of step S3, the information processor 20 causes the distance sensor 1 1 to detect a distance L again (step S1). As described above, while the distance L does not fall within the range from L1 to L2, the information processor 20 performs a loop process of repeating steps S1, S2, and S3 in this order.

On the other hand, when the distance L falls within the range from L1 to L2 (step S2; Yes), the notifier 22 of the information processor 20 performs detection enabled notification (step S4). Subsequently, the information processor 20 determines whether or not an operation input to start detection is performed (step S5). When no operation input to start detection has been performed (step S5; No), the information processor 20 causes the distance sensor 11 to detect a distance L again (step S1). As described above, while the distance L falls within the range from L1 to L2 (step S2; Yes) and no operation input to start detection has been performed (step S5; No), the information processor 20 repeats a loop process of repeating steps S1, S2, S4, and S5 in this order. When the distance L falls outside the range from L1 to L2 during the repetition (step S2; No), the information processor 20 returns to the loop process of performing steps S1, S2, and S3 in this order. Subsequently, when the distance L returns to a value within the range from L1 to L2 again (step S2; Yes), the information processor 20 is brought into a state of waiting for an operation input to start detection (step S5; No) and repeats the loop process of performing steps S1, S2, S4, and S5 in this order.

When an operation input to start detection is performed (step S5; Yes), the information processor 20 causes the substance sensor 10 to start taking in gas (step S6). Succeedingly, the information processor 20 determines whether or not a time period T that is set in advance as a time period required to take in the gas has elapsed (step S7). When while the time period T has not elapsed (step S7; No), the distance L falls within the range from L1 to L2 (step S2; Yes), the information processor 20 causes the substance sensor 10 to continue taking in the gas (step S6). That is, the information processor 20 performs a loop process of repeating steps S1, S2, S4, S5, S6, and S7 in this order and takes in the gas only for the predetermined time period T. When the distance L falls outside the range from L1 to L2 (step S2; No), the information processor 20 performs the loop process of repeating steps S1, S2, and S3 in this order again. When the distance L returns to a value within the range from L1 to L2 again (step S2; Yes), the information processor 20 repeats steps S1, S2, S4, S5, S6, and S7 in this order. The time period T can be set as an accumulated time period during which the gas is taken in. Note that the time period T may be initialized every time the distance L returns to a value within the range from L1 to L2.

When the time period T has elapsed (step S7; Yes), the information processor 20 causes the substance sensor 10 to detect a substance 3 (step S8). In this step, the information processor 20 inputs an electrical signal indicating a detection level of the substance 3 that is output from the substance sensor 10 and detects the substance 3, based on the input electrical signal. Although a detection result of the substance 3 is output to the outside, it may the detection result is stored inside the information processor 20. After completion of step S8, the substance detection processing is terminated.

Note that it may be configured to immediately detect the substance 3 without waiting for elapse of the time period T. In this case, when the distance L falls within the range from L1 to L2 (step S2; Yes), the information processor 20 causes the substance sensor 10 to detect the substance 3 (step S8). After completion of step S8, the information processor 20 terminates the substance detection processing.

The detection level of the substance sensor 10 is influenced by the distance L from the generation source 2. This is because as the distance L from the generation source 2 increases, concentration of the substance 3 gradually becomes lower. The substance detection system 1A according to the present embodiment detects the substance 3 only when the distance L between the substance sensor 10 and the generation source 2 of the substance 3 falls within the range of the distance L1 or more and the distance L2 or less.

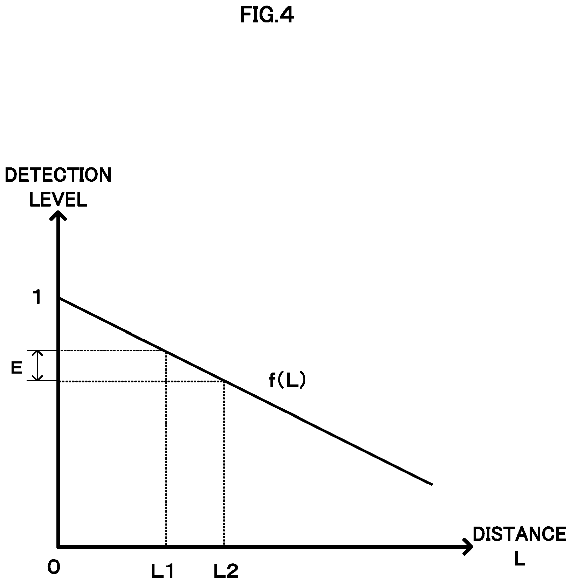

In FIG. 4, a relationship between the distance L and the detection level of the substance 3 in the substance sensor 10 is illustrated. In the graph, the detection level is normalized in such a manner that the detection level has a value of 1 when the distance L is 0. When the detection of the substance 3 is performed regardless of magnitude of the distance L, the detection level of the substance 3 is largely influenced by the distance L at the time of detection, as illustrated in FIG. 4. In contrast, when the distance L at the time of detection of the substance 3 is brought to a value within the range of the distance L1 or more and the distance L2 or less, variation in the detection level according to the distance L can be controlled to approximately a width E. This relationship indicates that limiting the distance L when the substance 3 is detected enables variation in the detection result of the substance 3 due to variation in the distance L to be reduced.

Note that in FIG. 4, a relationship between the distance L and the detection level of the substance 3 is illustrated as a linear relationship. However, the distance L and the detection level of the substance 3 does not always have a linear relationship. Although since the detection level of the substance 3 is closely related to the concentration of the substance 3, the detection level decreases as the distance L increases, change in the detection level is sometimes nonlinear. The characteristics can be expressed by a monotonically decreasing function f(L). That is, f(L) can be a nonlinear function.

As the range from L1 to L2 where a detection result of the substance sensor 10 is determined to be valid, a range where some variation in the detection level is allowed can be selected. For example, in the above-described function f(L), a range where variation in the detection level is small can be determined as the range from L1 to L2. In this case, it is possible to obtain actual measurement data of the detection level of the substance 3 with respect to the distance L in advance by detecting the substance 3 emitted from the generation source 2 by the substance sensor 10 while changing the distance L, calculate the function f(L), based on the actual measurement data, and determine the range from L1 to L2, based on the calculated function f(L).

The function f(L) can be used by performing statistical processing based on the actual measurement data. For example, the function f(L) can be calculated using a least-square method or the like. In addition, by performing machine learning, in particular deep learning, the function f(L) can be calculated or the range from L1 to L2 where variation in the detection result of the substance 3 is smallest can be directly calculated.

Embodiment 2

Embodiment 2 of the present disclosure is described. The substance detection system 1A according to Embodiment 1 described above notifies the user that the substance 3 can be detected when the distance L between the substance sensor 10 and the generation source 2 falls within the range from L1 to L2 and performs detection of the substance 3 by the substance sensor 10 caused by an operation input from the user. In contrast, a substance detection system according to Embodiment 2 automatically performs detection of a substance 3 when distance L between a substance sensor 10 and a generation source 2 falls within a range from L1 to L2.

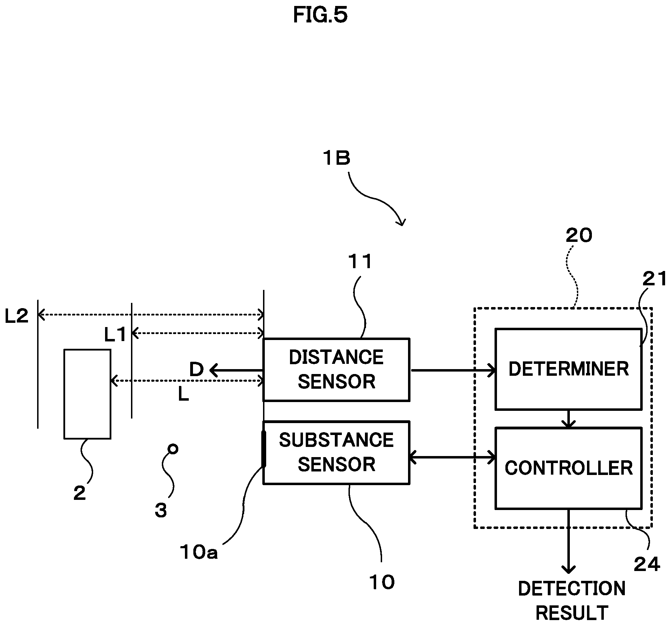

As illustrated in FIG. 5, a substance detection system 1B according to the present embodiment is the same as the substance detection system 1A in that the substance detection system 1B includes a substance sensor 10, a distance sensor 11, and an information processor 20. In the substance detection system 1B, the information processor 20 includes a determiner 21 and a controller 24.

Operation of the determiner 21 is the same as the operation of the determiner 21 in Embodiment 1 described above. That is, the determiner 21 determines whether or not the distance L detected by the distance sensor 11 falls within a range from L1 to L2 where a detection result by the substance sensor 10 is determined to be valid.

The controller 24 starts detection of the substance 3 by the substance sensor 10 when the determiner 21 determines that the distance L detected by the distance sensor 11 falls within the range from L1 to L2. On the other hand, the controller 24 suspends the detection of the substance 3 by the substance sensor 10 when the determiner 21 determines that the distance L detected by the distance sensor 11 falls outside the range from L1 to L2.

Next, operation of the substance detection system 1B according to the present embodiment of the present disclosure is described. The following description is made mainly on substance detection processing that is performed by the information processor 20.

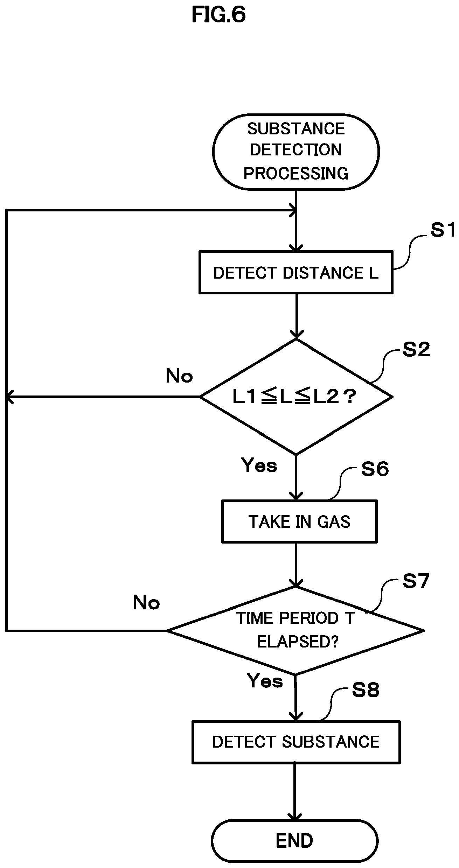

As illustrated in FIG. 6, first, the information processor 20 causes the distance sensor 11 to detect a distance L to the generation source 2 (step S1). Succeedingly, the determiner 21 of the information processor 20 determines whether or not the distance L detected by the distance sensor 11 falls within the range from L1 to L2, that is, $L1 \leq L \leq L2$ (step S2). When the distance L does not fall within the range from L1 to L2 (step S2; No), the information processor 20 causes the distance sensor 11 to detect a distance L again (step S 1). As described above, while the distance L does not fall within the range from L1 to L2, the information processor 20 performs a loop process of repeating steps S 1 and S2 in this order.

On the other hand, when the distance L falls within the range from L1 to L2 (step S2; Yes), the information processor 20 causes the substance sensor 10 to start taking in gas (step S6). Succeedingly, the information processor 20 determines whether or not a time period T that is set in advance as a time period required to take in the gas has elapsed (step S7). When while the time period T has not elapsed (step S7; No), the distance L falls within the range from L1 to L2 (step S2; Yes), the information processor 20 causes the substance sensor 10 to continue taking in the gas (step S6). That is, the information processor 20 performs a loop process of repeating steps S1, S2, S6, and S7 in this order. When the distance L falls outside the range from L1 to L2 (step S2; No), the information processor 20 performs the loop process of repeating steps S 1 and S2 in this order again. Subsequently, when the distance L returns to a value within the range from L1 to L2 (step S2; Yes), the information processor 20 repeats steps S1, S2, S6, and S7 in this order again and continues taking in the gas.

When the time period T has elapsed (step S7; Yes), the information processor 20 causes the substance sensor 10 to detect a substance 3 (step S8). Specifically, the information processor 20 inputs an electrical signal indicating a detection level of the substance 3 that is output from the substance sensor 10 and detects the substance 3, based on the input signal. A detection result is output to the outside. After completion of step S8, the substance detection processing is terminated.

As described above, the detection of the substance 3 can be performed without performing notification to the outside when the distance L falls within the range from L1 to L2.

In the present embodiment, it may also be configured to immediately detect the substance 3 without waiting for elapse of the time period T. In this case, when the distance L falls within the range from L1 to L2 (step S2; Yes), the information processor 20 causes the substance sensor 10 to detect the substance 3 (step S8). After completion of step S8, the information processor 20 terminates the substance detection processing.

Embodiment 3

Next, Embodiment 3 of the present disclosure is described. In the substance detection systems 1A and 1B according to Embodiments 1 and 2 described above, the number of types of generation sources 2 to be detected is basically one and the number of ranges from L1 to L2 where detection is valid is one. In contrast, a substance detection system according to Embodiment 3 deals with a plurality of types of generation sources 2. In general, when types of generation sources 2 are different from one another, ranges from L1 to L2 where detection is valid also differ from one another. Thus, in Embodiment 3, a range from L1 to L2 is changed with respect to each generation source 2 to be detected.

In the substance detection system according to Embodiment 3, a substance sensor 10 is capable of detecting substances 3 emitted from a plurality of types of generation sources 2, such as a generation source A, a generation source B, a generation source C, and so on.

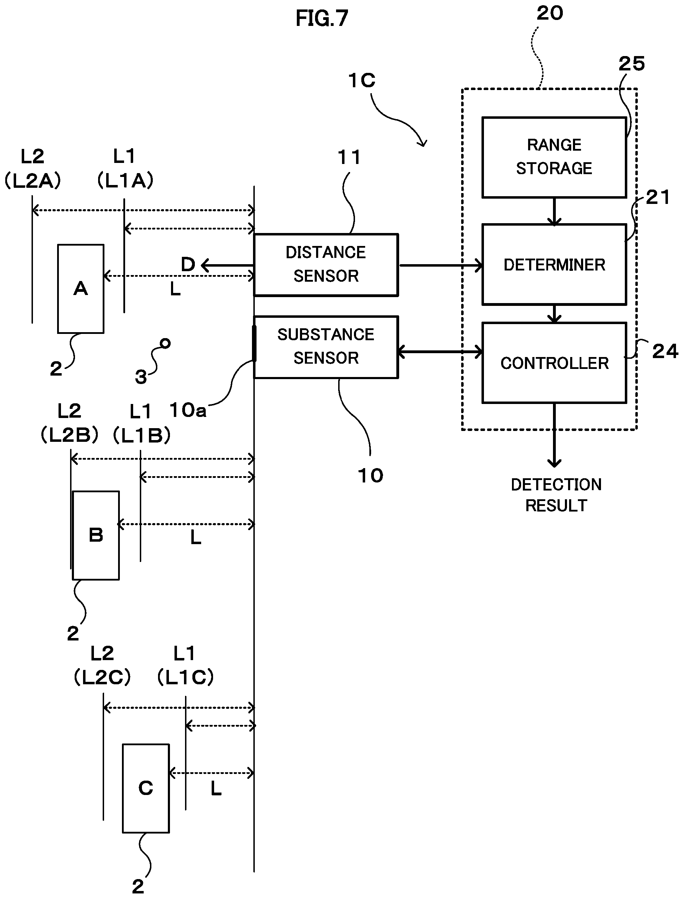

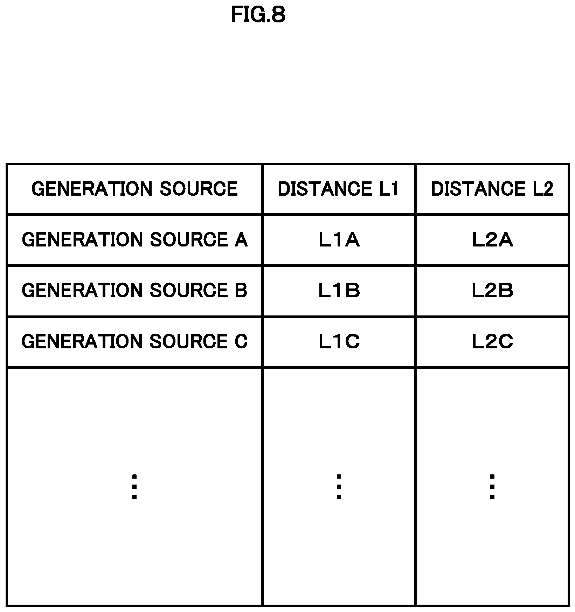

As illustrated in FIG. 7, an information processor 20 constituting a substance detection system 1C includes a range storage 25 that stores a different range from L1 to L2 with respect to each generation source 2 to be detected. As illustrated in FIG. 8, in the range storage 25, a generation source 2 and distances L1 and L2 are stored in association with each other. For example, when it is assumed that a distance L1 and a distance L2 corresponding to the generation source A are denoted by L1A and L2A, respectively, the distances L1A and L2A are stored in association with the generation source A. L1A and L2A represent specific numerical values. The same applies to distances L1B and L2B corresponding to the generation source B and distances L1C and L2C corresponding to the generation source C.

A determiner 21 reads a range from L1 to L2 corresponding to a substance 3 emitted from a generation source 2 serving as a detection target from the range storage 25. The determiner 21 performs determination of the distance L with the read range from L1 to L2 used as criteria. Reading of a range from L1 to L2 is performed every time a generation source 2 to be detected is changed. Specifically, the determiner 21 uses the range from L1A to L2A as determination criteria for the generation source A, uses the range from L1B to L2B as determination criteria for the generation source B, and uses the range from L1C to L2C as determination criteria for the generation source C. Change of a generation source 2 serving as a detection target is performed by an operation input from a user.

When configured in such a manner, it becomes possible to detect a substance 3 in an appropriate range from L1 to L2 with respect to each generation source 2 serving as a detection target.

Embodiment 4

Embodiment 4 of the present disclosure is described. In the substance detection systems 1A to 1C according to Embodiments 1 to 3 described above, a detection result of a substance 3 is output to the outside. A substance detection system according to Embodiment 4 stores a detection result of a substance 3 in association with a detection result by a distance sensor 11.

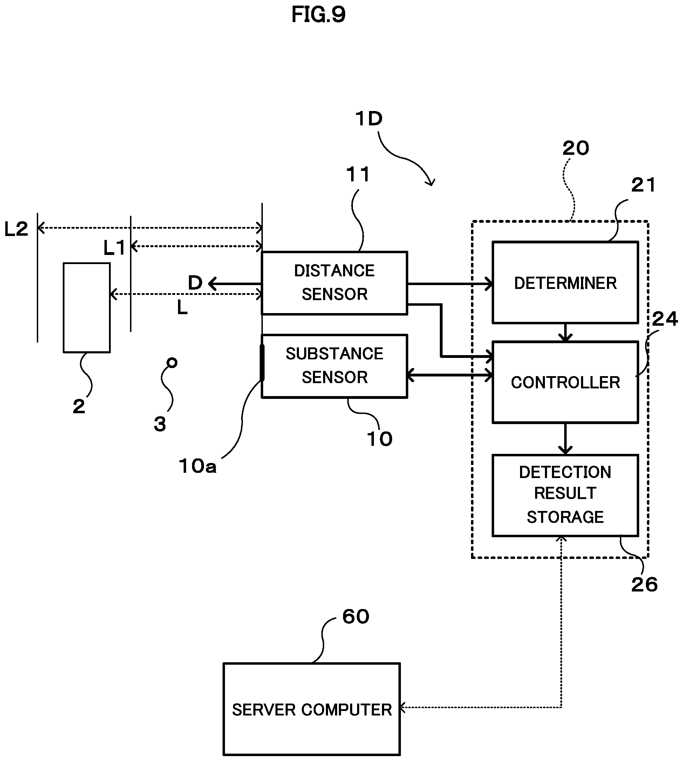

As illustrated in FIG. 9, in a substance detection system 1D according to Embodiment 4, an information processor 20 includes, in addition to a determiner 21 and a controller 24, a detection result storage 26 that stores a detection result by a substance sensor 10 and a detection result by the distance sensor 11 in association with each other.

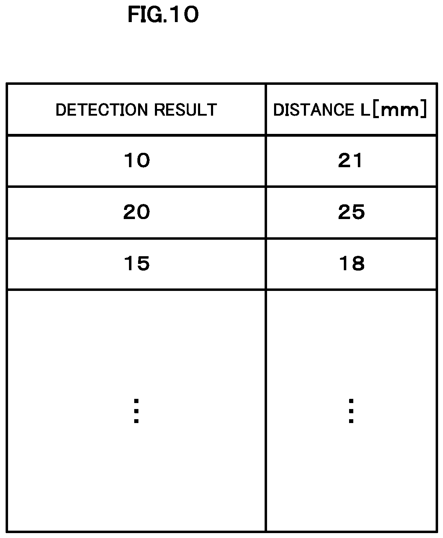

As illustrated in FIG. 10, in the detection result storage 26, a detection result of the substance 3 by the substance sensor 10 and a distance L detected by the distance sensor 11 are stored in association with each other. The substance detection system 1D according to the present embodiment can be used to investigate a relationship between a detection result of the substance 3 and the distance L.

In Embodiment 4, two operation modes, namely a data acquisition mode and a detection mode, are prepared as operation modes of the substance detection system 1D.

In the data acquisition mode, the substance detection system 1D is in a state in which no range from L1 to L2 has been set. When the substance detection system 1D is in this state, the information processor 20 of the substance detection system 1D causes the controller 24 to control the distance sensor 11 to detect a distance L to a generation source 2 and to also control the substance sensor 10 to detect the substance 3 without causing the determiner 21 to perform determination. Detection results of the distance L and the substance 3 are stored in the detection result storage 26.

Data stored in the detection result storage 26 are, for example, sent to a server computer 60 via a communication network and are collected by the server computer 60. The server computer 60 performs machine learning using collected data as training data and thereby determines a range from L1 to L2 corresponding to the generation source 2. The server computer 60 transmits the determined range from L1 to L2 to the substance detection system 1D, and the substance detection system 1D stores the determined range from L1 to L2. A range from L1 to L2 is determined with respect to each type of generation source 2. That is, as illustrated in FIG. 8, distances L1A and L2A are determined with respect to a generation source A, distances L1B and L2B are determined with respect to a generation source B, and distances L1C and L2C are determined with respect to a generation source C.

Subsequently, the substance detection system 1D transitions to the detection mode and performs detection of the substance 3 by the substance sensor 10 when the distance L to the generation source 2 that is detected by the distance sensor 11 falls within the range from L1 to L2.

In addition, according to the substance detection system 1D, a user can, for example, grasp whether a detection result of the substance 3 is obtained while the substance detection system 1D is located away from the generation source 2 or obtained while the substance detection system 1D is in proximity to the generation source 2. In addition, storing a detection result and a distance L in association with each other enables processing of correcting a detection level of the substance 3, based on a distance L detected by the distance sensor 11 afterward, or the like to be performed.

In addition, it may be configured to detect the substance 3 by the substance sensor 10 regardless of the distance L and store the distance L and a detection result of the substance 3 in association with each other in the detection result storage 26. In this case, it may be configured to delete a detection result when the substance detection system 1D is located outside the range from L1 to L2 among data stored in the detection result storage 26.

Embodiment 5

Embodiment 5 of the present disclosure is described. The substance detection systems 1A to 1D according to Embodiments 1 to 4 described above detect the substance 3 by the substance sensor 10 when the distance L to the generation source 2 falls within the range from L1 to L2. In contrast, a substance detection system according to Embodiment 5 corrects a detection result of a substance 3, based on distance L to a generation source 2 that is detected by a distance sensor 11.

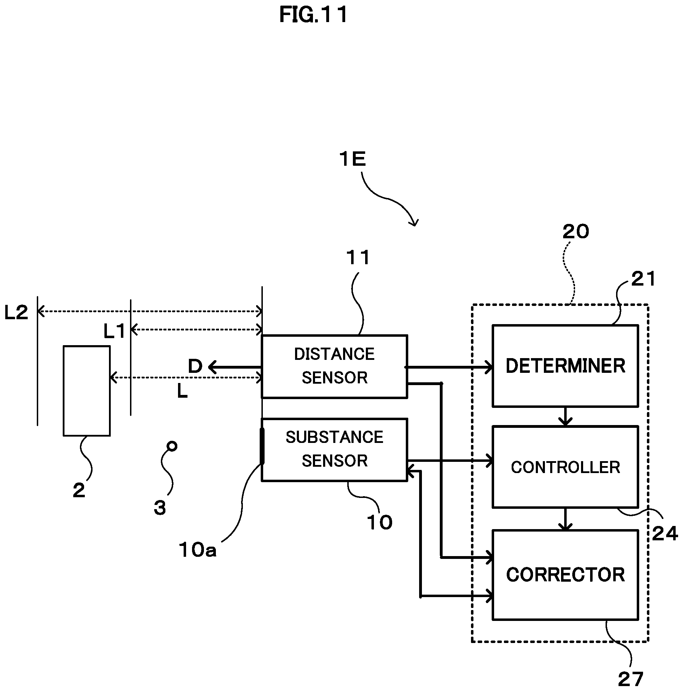

As illustrated in FIG. 11, in a substance detection system 1E according to Embodiment 5, an information processor 20 includes a corrector 27 as a first corrector. The corrector 27 corrects a detection result by a substance sensor 10, based on a detection result by the distance sensor 11.

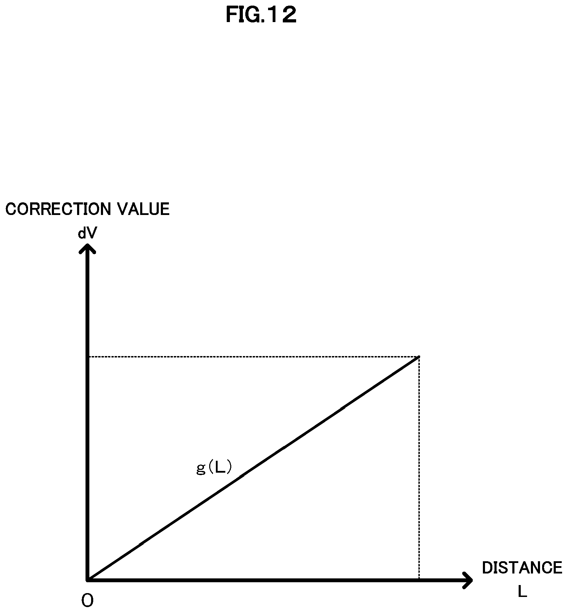

In FIG. 12, a relationship between the distance L detected by the distance sensor 11 and a correction value dV is illustrated. As illustrated in FIG. 12, the larger the distance L becomes, the larger the correction value dV becomes. The relationship between the distance L and the correction value dV can be expressed by the following equation.

$$\text{Correction value } dV = g(L)$$

That is, the correction value dV is a function g(L) of the distance L.

It is assumed that a detected value of the substance 3 detected by the substance sensor 10 is denoted by V. The detected value V can be corrected by use of, for example, the following conversion equation. It is assumed that a detected value of the substance 3 after correction is denoted by VL.

$$VL = V + dV$$

Note that in FIG. 12, the function g(L) is assumed to be a function in which the correction value dV linearly changes with respect to the distance L. However, the function g(L) does not have to be a linear function and may be a nonlinear function. The function g(L) can be calculated based on, for example, the amount of change in a function f(L) of the distance L that is illustrated in FIG. 4.

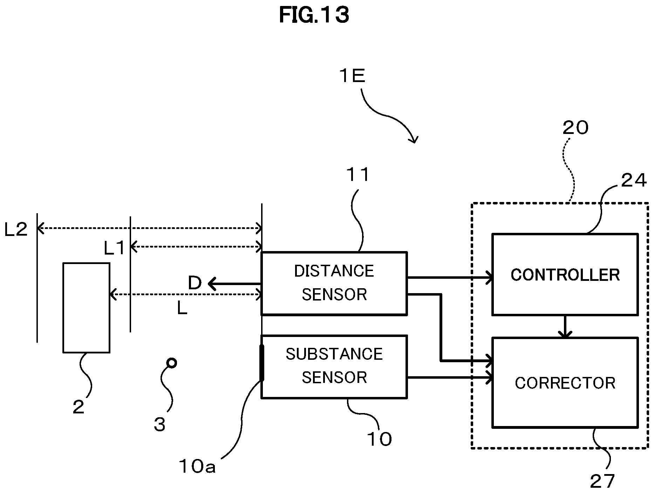

In addition, as illustrated in FIG. 13, the substance detection system 1E according to the present embodiment does not have to include a determiner 21. That is, in the present embodiment, determination of the distance L by the determiner 21 is not performed and even when the distance L falls outside a range from L1 to L2, the substance 3 can be detected by the substance sensor 10.

Embodiment 6

Embodiment 6 of the present disclosure is described. The substance detection systems 1A to 1E according to Embodiments 1 to 5 described above use a detected value of the substance 3 detected by the substance sensor 10 as a detected value of the substance 3 as it is. In contrast, a substance detection system according to Embodiment 6 corrects a detection level of a substance 3 according to an ambient environment, such as temperature and humidity.

As described above, a substance sensor 10 detects a change in weight of a sensitive film caused by adsorption of the substance 3 on the sensitive film. An adsorption state of the substance 3 on the sensitive film is influenced by the ambient environment, such as steam contained in gas. The substance detection system according to the present embodiment detects information about an ambient environment that influences the adsorption of the substance 3 on the sensitive film and corrects a detected value of the substance 3 detected by the substance sensor 10 by the detected environmental information.

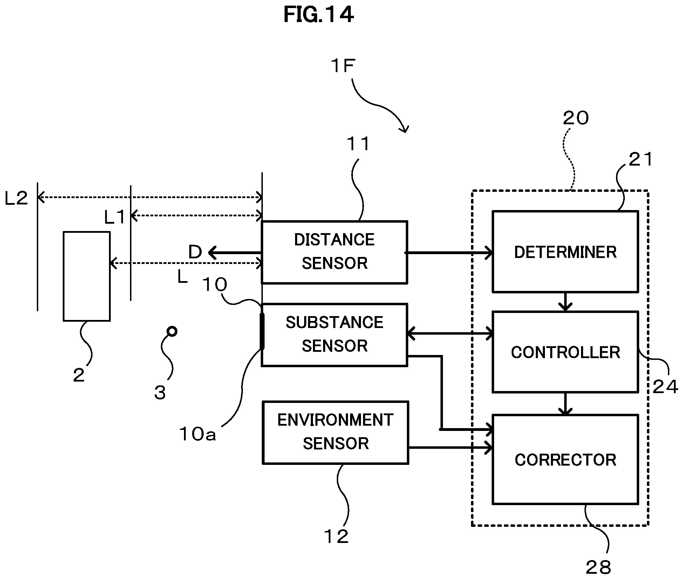

As illustrated in FIG. 14, a substance detection system 1F according to the present embodiment includes an environment sensor 12. The environment sensor 12 detects information about an ambient environment. Environmental information to be detected can be configured to be temperature or humidity that has a correlation with the amount of steam contained in ambient air.

In the substance detection system 1F according to the present embodiment, an information processor 20 includes a corrector 28 as a second corrector. The corrector 28 corrects a detection result by the substance sensor 10, based on a detection result by the environment sensor 12.

The corrector 28 stores a reference value for the environmental information. Further, the corrector 28 stores data representing a correspondence relation between a difference between environmental information detected by the environment sensor 12 and the reference value and a correction value of a detection level of the substance 3 detected by the substance sensor 10. The data may be, for example, a function that when a difference is input, outputs a correction value or a data group each element of which represents a correspondence relation between a difference and a correction value.

The corrector 28 calculates a difference between environmental information detected by the environment sensor 12 and the reference value. The corrector 28 calculates a correction value corresponding to the difference from the data representing a correspondence relation between a difference and a correction value. The corrector 28 corrects a detected value of the substance 3 detected by the substance sensor 10 by the correction value.

Note that the environmental information detected by the environment sensor 12 is not limited to temperature and humidity. The environment sensor 12 may be configured to directly detect the amount of steam contained in the gas. In addition, an environment sensor to detect the amount of substance that is a substance contained in the gas and promotes or inhibits adsorption of the substance 3 on the sensitive film can be used. That is, the environment sensor 12 is only required to be a sensor that detects environmental information influencing a detection level of the substance 3 detected by the substance sensor 10.

The correction based on a detection result by the environment sensor 12 can be combined with the correction based on a detection result by the distance sensor 11. It may a detected value of the substance 3 detected by the substance sensor 10 is corrected by environmental information detected by the environment sensor 12 and the detected value corrected by the environmental information is further corrected by the distance L detected by the distance sensor 11. Whether a detected value by the substance sensor 10 is first corrected by a detection result by the environment sensor 12 or is first corrected by a detection result by the distance sensor 11 can be appropriately determined. In addition, it may output of a single function that has a detection result by the environment sensor 12 and a detection result by the distance sensor 11 as arguments is used as a correction value.

Embodiment 7

Embodiment 7 of the present disclosure is described. In Embodiments 1 to 6 described above, functional internal structures of the substance detection systems are described. In Embodiment 7, a physical structure of a substance detection system is described.

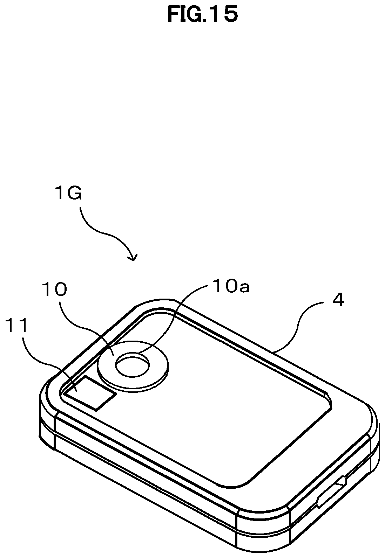

As illustrated in FIG. 15, an external appearance of a substance detection system 1G according to the present embodiment is flat plate-shaped as a whole, and a substance sensor 10 and a distance sensor 11 are disposed on the same surface. An intake port 10a of the substance sensor 10 faces in a direction in which the distance sensor 11 detects distance L, and the substance sensor 10 and the distance sensor 11 are closely arranged to the extent that the distance L detected by the distance sensor 11 can be considered equal to a distance between the substance sensor 10 and a generation source 2.

In the substance detection system 1G, the substance sensor 10 and the distance sensor 11 are housed in the same housing 4 and are integrated with each other. An information processor 20 is housed in the housing 4. When configured in such a manner, an entire system can be made compact.

Note that an environment sensor 12 (not illustrated) may be configured to be integrated with the substance sensor 10 and the distance sensor 11. The substance sensor 10, the distance sensor 11, and the environment sensor 12 may be configured to be attachable and detachable.

Embodiment 8

Embodiment 8 of the present disclosure is described. In the substance detection system 1G according to Embodiment 7 described above, the substance sensor 10 and the distance sensor 11 are integrated with each other. In contrast, in a substance detection system according to the present embodiment, each of a substance sensor 10 and a distance sensor 11 is housed in a separate unit.

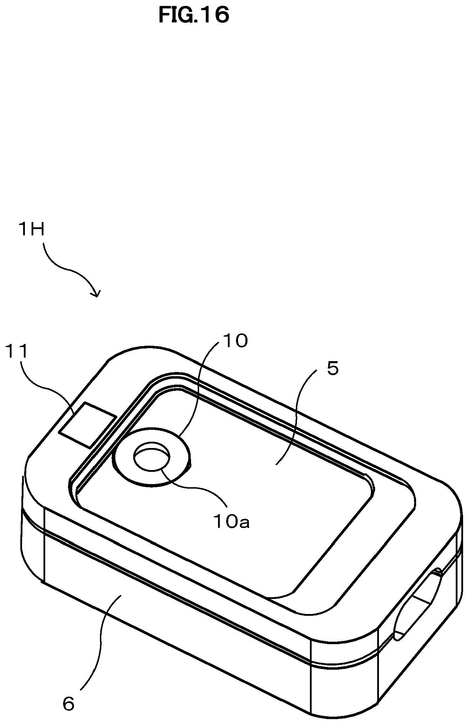

Specifically, as illustrated in FIG. 16, a substance detection system 1H according to the present embodiment includes a unit 5 and a unit 6. In the unit 5, the substance sensor 10 is housed, and in the unit 6, the distance sensor 11 is housed. An information processor 20 is housed in the unit 5 or the unit 6 or is housed in both the units 5 and 6.

The unit 6 is formed in a shape surrounding the unit 5 and is attached to the unit 5. When the unit 6 is attached to the unit 5, an intake port 10a of the substance sensor 10 is brought into a state of facing in a direction in which the distance sensor 11 detects distance L, and the distance L detected by the distance sensor 11 is brought into a state of being able to be considered equal to a distance L between the substance sensor 10 and a generation source 2.

As described above, in the substance detection system 1H according to the present embodiment, the substance sensor 10 and the distance sensor 11 can be separated from each other. When configured in such a manner, even in the case where either the substance sensor 10 or the distance sensor 11 malfunctions, the unit 6 including the distance sensor 11 or the unit 5 including the substance sensor 10 can be easily replaced.

Note that when the substance detection system 1H includes an environment sensor 12, the environment sensor 12 may be housed in the unit 5 or may be housed in the unit 6. In addition, the environment sensor 12 may be configured to be housed in another unit that differs from the units 5 and 6 and is attachable and detachable to and from the units 5 and 6.

Embodiment 9

Embodiment 9 of the present disclosure is described. In the substance detection systems 1G and 1H according to Embodiments 7 and 8 described above, the substance sensor 10 and the distance sensor 11 are configured to be housed in the housing 4 or the units 5 and 6, respectively, in which the information processor 20 is housed. In contrast, in a substance detection system according to the present embodiment, a substance sensor 10 and a distance sensor 11 are housed in an adapter that is separated from a main body in which an information processor 20 is housed.

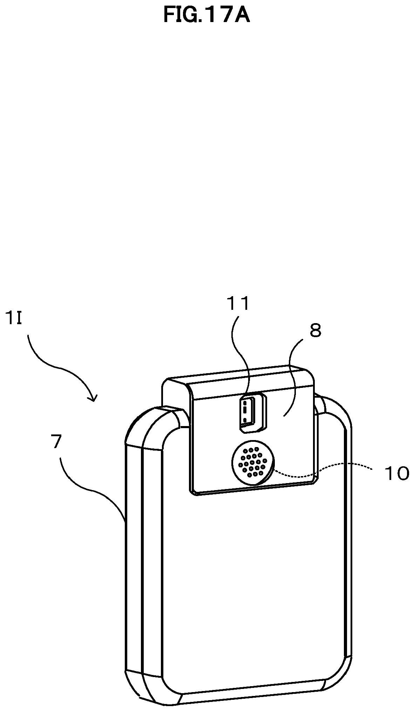

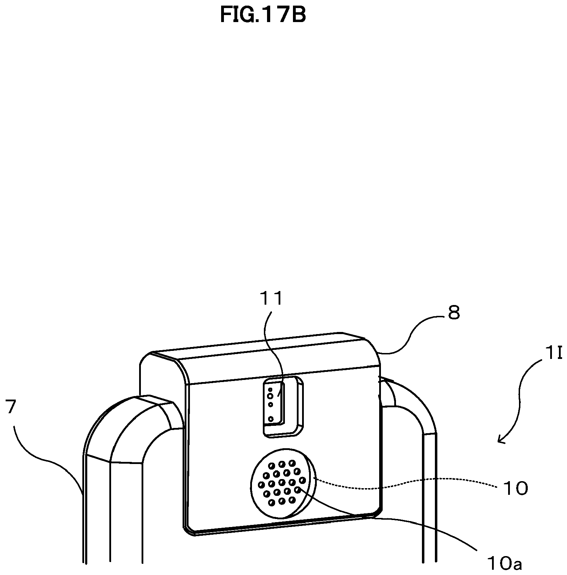

As illustrated in FIGS. 17A and 17B, a substance detection system 1I according to the present embodiment includes a main body 7 and an adapter 8 that is attachable and detachable to and from the main body 7. The substance sensor 10 and the distance sensor 11 are installed in the adapter 8. An intake port 10a of the substance sensor 10 is in a state of facing in a direction in which the distance sensor 11 measures distance L. In addition, the substance sensor 10 and the distance sensor 11 are closely arranged in such a way that the distance L detected by the distance sensor 11 can be considered equal to a distance L between the substance sensor 10 and a generation source 2.

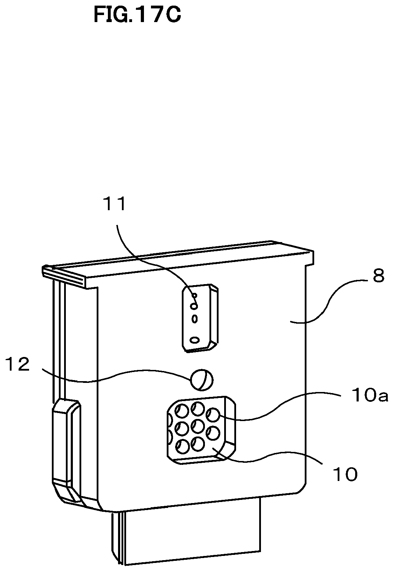

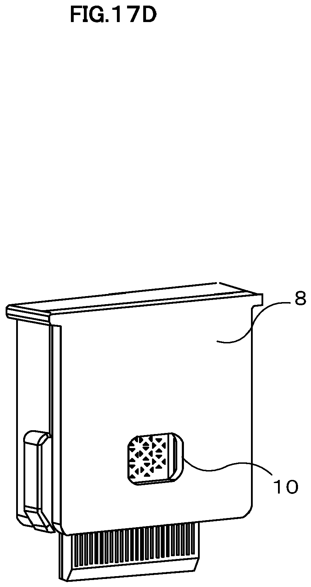

In FIGS. 17C and 17D, the adapter 8 from which a cover is removed is illustrated. As illustrated in FIGS. 17C and 17D, in addition to the substance sensor 10 and the distance sensor 11, an environment sensor 12 is disposed in the adapter 8. The environment sensor 12 detects information about an ambient environment around the substance sensor 10. In addition, the information processor 20 is installed in the main body 7.

An adapter 8 for each generation source 2, that is, a plurality of adapters 8, is prepared. In this case, the type of substance 3 to be detected by a substance sensor 10 mounted on an adapter 8 is different for each adapter 8. For example, an adapter 8 for detection of a generation source A, an adapter 8 for detection of a generation source B, and an adapter 8 for detection of a generation source C are individually prepared. When the generation source A is a detection target, the adapter 8 for detection of the generation source A is used by being attached to the main body 7.

When configured in such a manner, only replacing the adapter 8 enables a generation source 2 to be detected to be easily changed.

As described in the foregoing, the substance detection systems 1A to 1I according to the above-described embodiments include the determiner 21 to determine whether or not the distance L between the generation source 2 and the substance sensor 10 falls within a range where a detection result by the substance sensor 10 is determined to be valid. Thus, a detection result by the substance sensor 10 when the above-described distance L falls within the range from L1 to L2 can be determined to be valid. As a result, precision of the detection result can be stabilized.

In addition, the substance detection system 1A according to Embodiment 1 described above includes the notifier 22 to notify that the substance 3 can be detected by the substance sensor 10 when the determiner 21 determines that the distance L detected by the distance sensor 11 falls within the range from L1 to L2. When configured in such a manner, it becomes possible for a user who uses the substance detection system 1A to become aware that the substance detection system 1A is in a state in which the distance L is determined to be appropriate and to detect the substance 3 while the substance detection system 1A is in the state.

In addition, according to the substance detection system 1A according to Embodiment 1 described above, the notifier 22 notifies that the substance 3 cannot be detected by the substance sensor 10 when the determiner 21 determines that the distance L detected by the distance sensor 11 falls outside the range from L1 to L2. When configured in such a manner, it becomes possible for the user who uses the substance detection system 1A to become aware that the distance L falls outside an appropriate range and to be prompted to return the distance L to within the range.

In addition, the substance detection system 1B according to Embodiment 2 described above includes the controller 24 to start detection of the substance 3 by the substance sensor 10 when the determiner 21 determines that the distance L detected by the distance sensor 11 falls within the range from L1 to L2. When configured in such a manner, it becomes possible to automatically detect the substance 3 only when the distance L falls within an appropriate range from L1 to L2.

In addition, according to the substance detection system 1B according to Embodiment 2 described above, the controller 24 suspends detection of the substance 3 by the substance sensor 10 when the determiner 21 determines that the distance L detected by the distance sensor 11 falls outside the range from L1 to L2. When configured in such a manner, it becomes possible to automatically suspend detection of the substance 3 when the distance L falls outside the range from L1 to L2.

In addition, the substance detection system 1C according to Embodiment 3 described above includes the range storage 25 to store a range from L1 to L2 with respect to each generation source 2 serving as a detection target. The determiner 21 performs determination by reading a range from L1 to L2 corresponding to a generation source 2 from the range storage 25. When configured in such a manner, it becomes possible to detect a substance 3 in an appropriate range from L1 to L2 that is different for each generation source 2.

In addition, the substance detection system 1D according to Embodiment 4 described above includes the detection result storage 26 to store a detection result by the substance sensor 10 and a detection result by the distance sensor 11 in association with each other. When configured in such a manner, a relationship between a detection result by the substance sensor 10 and the distance L to the generation source 2 can be clarified.

In addition, the substance detection system 1E according to Embodiment 5 described above includes the substance sensor 10 to detect the substance 3 emitted from the generation source 2, the distance sensor 11 to detect the distance L between the generation source 2 and the substance sensor 10, and the corrector 27 to correct a detection result by the substance sensor 10, based on a detection result by the distance sensor 11. When configured in such a manner, influence of the distance L on a detection result of the substance 3 can be eliminated and the substance 3 can be detected with high precision.

In addition, the substance detection system 1F according to Embodiment 6 described above includes the environment sensor 12 to detect information about an ambient environment. The corrector 28 corrects a detection result by the substance sensor 10, based on a detection result by the environment sensor 12. When configured in such a manner, it becomes possible to detect the substance 3 without being influenced by an ambient environment.

In addition, according to the substance detection system 1G according to Embodiment 7 described above, the substance sensor 10 and the distance sensor 11 are integrated with each other. When configured in such a manner, since a positional relationship between the substance sensor 10 and the distance sensor 11 can be fixed, a detection result of the substance 3 can be acquired always under the same conditions.

In addition, according to the substance detection system 1H according to Embodiment 8 described above, the substance sensor 10 and the distance sensor 11 can be configured to be separable from each other. When configured in such a manner, in the case where, for example, the distance sensor 11 malfunctions, the distance sensor 11 can be easily replaced.

In addition, according to the substance detection system 1I according to Embodiment 9 described above, the substance detection system 1I includes the main body 7 and the adapter 8 attachable and detachable to and from the main body 7 and the substance sensor 10 and the distance sensor 11 are installed in the adapter 8. When configured in such a manner, only replacing the adapter 8 enables a generation source 2 serving as a detection target to be easily changed.

In addition, as the substance detection system 1G illustrated in FIG. 15, the environment sensor 12 may be an environment sensor integrated with the substance sensor 10 and the distance sensor 11. In addition, the environment sensor 12 may be an environment sensor that is installed in a unit separated from a unit in which the substance sensor 10 is installed and a unit in which the distance sensor 11 is installed. Further, at least two of the substance sensor 10, the distance sensor 11, and the environment sensor 12 may be configured to be separable from each other.

Note that in the above-described embodiment, the notifier 22 performs both the detection enabled notification when the distance L falls within the range from L1 to L2 and the detection disabled notification when the distance L falls outside the range from L1 to L2. However, the present disclosure is not limited to the configuration. The notifier 22 may perform only one of the detection enabled notification and the detection disabled notification.

Note that in the above-described embodiments, it is assumed that the substance sensor 10 is a sensor to detect the substance 3 based on a change in vibration frequency of an adsorption film. However, the present disclosure is not limited to the configuration. For example, the substance sensor 10 may be a sensor that detects adsorption of the substance 3 on the adsorption film by detecting a change in current flowing to the adsorption film, voltage across the adsorption film, or resistance of the adsorption film or a change in capacitance of the adsorption film or may be a sensor that detects adsorption of the substance 3 on the adsorption film based on another principle. The present disclosure is not limited to a specific detection method of the substance 3.

Note that the substance detection systems 1A to 1I can be used to identify the generation source 2 of the substance 3. For example, when a generation source 2 of some substance 3 is unclear, the distance sensor 11 is pointed toward an object that is possibly the generation source 2 and the substance 3 is detected by the substance sensor 10 while distance to the object is changed. The generation source 2 can be identified based on whether or not a detection level of the substance 3 increases as the substance sensor 10 is brought closer to the substance 3 and the detection level of the substance 3 decreases as the substance sensor 10 is kept further away from the substance 3. Note that in the case of the substance detection system 1D according to Embodiment 4 described above, it may a mode in which the substance detection system 1D operates in such a manner as to search for a generation source 2 is set as a search mode, the substance detection system 1D is configured to be able to operate in three operation modes, namely the search mode, the data acquisition mode, and the detection mode, and the substance detection system 1D, after identifying a generation source 2 in the search mode, transitions to the data acquisition mode and determines a range from L1 to L2, transitions to the detection mode, and when the distance L falls within the range from L1 to L2, detects the substance 3 by the substance sensor 10.

As described above, the hardware configurations and the software configurations of the substance detection systems 1A to 1I are only an example and can be arbitrarily changed and modified.

A central part performing processing of the substance detection systems 1A to 1I each of which includes the CPU 30, the memory 31, the external storage 32, the input/output device 33, the card interface 34, the communication interface 35, the operation input device 36, the display 37, the internal bus 40, and the like can be achieved using a general computer system instead of a dedicated system. The computer system is, for example, a smartphone, a tablet, a mobile phone, a personal computer, a mobile computer, or the like. For example, the substance detection systems 1A to 1I that execute the above-described processing may be configured by storing and distributing computer programs for performing the above-described operation in a non-transitory computer-readable recording medium (such as a flexible disk, a CD-ROM, and a DVD-ROM) and installing the computer programs in the computer. In addition, the substance detection systems 1A to 1I may also be configured by storing the computer programs in a storage device that a server device on a communication network, such as the Internet, has and a general computer system downloading the computer programs.

When the functions of the substance detection systems 1A to 1I are to be achieved through sharing between an operating system (OS) and an application program or collaboration between the OS and the application program, only the application program part may be stored in a non-transitory recording medium or a storage device.

It is also possible to superimpose a computer program on a carrier wave and distribute the computer program via a communication network. For example, the computer program may be posted on a bulletin board system (BBS) on the communication network, and the computer program may be distributed via the network. The above-described processing may be configured to be able to be performed by starting up and executing the distributed computer program in a similar manner to other application programs under the control of the OS.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2021-131733, filed on August 12, 2021, the entire disclosure of which is incorporated by reference herein.

Industrial Applicability

The present disclosure can be applied to detection of a substance emitted from a generation source.

Reference Signs List

1A, 1B, 1C, 1D, 1E, 1F, 1G, 1H, 1I    Substance detection system

| | |
|---|---|
| 2 | Generation source |
| 3 | Substance |
| 4 | Housing |
| 5, 6 | Unit |
| 7 | Main body |
| 8 | Adapter |
| 10 | Substance sensor |
| 10a | Intake port |
| 11 | Distance sensor |
| 12 | Environment sensor |
| 20 | Information processor |
| 21 | Determiner |
| 22 | Notifier |
| 23, 24 | Controller |
| 25 | Range storage |
| 26 | Detection result storage |
| 27, 28 | Corrector |
| 30 | CPU |
| 31 | Memory |
| 32 | External storage |
| 33 | Input/output device |
| 34 | Card interface |
| 35 | Communication interface |
| 36 | Operation input device |
| 37 | Display |
| 39 | Program |
| 40 | Internal bus |
| 50 | Non-transitory recording medium |
| 60 | Server computer |

**Claims**

1. A substance detection system, comprising:

   a substance sensor to detect a substance emitted from a detection target;
   a distance sensor to detect a distance between the detection target and the substance sensor; and
   a determiner to determine whether or not distance detected by the distance sensor falls within a range where a detection result by the substance sensor is determined to be valid.

2. The substance detection system according to claim 1, comprising:
   a notifier to notify that a substance can be detected by the substance sensor when the determiner determines that distance detected by the distance sensor falls within the range.

3. The substance detection system according to claim 2, wherein the notifier notifies that a substance cannot be detected by the substance sensor when the determiner determines that distance detected by the distance sensor falls outside the range.

4. The substance detection system according to any one of claims 1 to 3, comprising:
   a controller to start detection of a substance by the substance sensor when the determiner determines that distance detected by the distance sensor falls within the range.

5. The substance detection system according to claim 4, wherein the controller suspends detection of a substance by the substance sensor when the determiner determines that distance detected by the distance sensor falls outside the range.

6. The substance detection system according to any one of claims 1 to 5, comprising:

   a range storage to store the range with respect to each of the detection targets, wherein
   the determiner performs determination by reading the range corresponding to the detection target from the range storage.

7. The substance detection system according to any one of claims 1 to 6, comprising:
   a detection result storage to store a detection result by the substance sensor and a detection result by the distance sensor in association with each other.

8. A substance detection system, comprising:

   a substance sensor to detect a substance emitted from a detection target;
   a distance sensor to detect a distance between the detection target and the substance sensor; and
   a first corrector to correct a detection result by the substance sensor, based on a detection result by the distance sensor.

9. The substance detection system according to any one of claims 1 to 8, comprising

   an environment sensor to detect information about an ambient environment; and
   a second corrector to correct a detection result by the substance sensor, based on a detection result by the environment sensor.

10. The substance detection system according to any one of claims 1 to 8, wherein the substance sensor and the distance sensor are integrated with each other.

11. The substance detection system according to claim 10, comprising:

    a main body; and
    an adapter attachable and detachable to and from the main body, wherein
    the substance sensor and the distance sensor are installed in the adapter.

12. The substance detection system according to claim 10, wherein the substance sensor and the distance sensor are separable from each other.

13. The substance detection system according to claim 9, wherein the substance sensor, the distance sensor, and the environment sensor are integrated with one another.

14. The substance detection system according to claim 13, comprising:

    a main body; and
    an adapter attachable and detachable to and from the main body, wherein
    the substance sensor, the distance sensor, and the environment sensor are installed in the adapter.

15. The substance detection system according to claim 13, wherein at least two of the substance sensor, the distance sensor, or the environment sensor are separable from each other.

FIG.1

# FIG.2

## FIG.3

```
        ┌─────────────────────────┐
        │  SUBSTANCE DETECTION    │
        │       PROCESSING        │
        └─────────────────────────┘
                     │
                     ▼              S1
        ┌─────────────────────────┐
        │    DETECT DISTANCE L    │
        └─────────────────────────┘
                     │
                     ▼              S2
   No            ╱─────────╲
  ◀─────────────  L1≦L≦L2 ?
                  ╲─────────╱
                     │ Yes
         S3          ▼          S4
  ┌──────────────┐      ┌──────────────┐
  │  DETECTION   │      │  DETECTION   │
  │   DISABLED   │      │   ENABLED    │
  │ NOTIFICATION │      │ NOTIFICATION │
  └──────────────┘      └──────────────┘
         │                     │
  ◀──────┘                     ▼          S5
   No                     ╱─────────╲
  ◀──────────────────────  DETECTION
                           STARTS?
                          ╲─────────╱
                              │ Yes      S6
                     ┌─────────────────┐
                     │   TAKE IN GAS   │
                     └─────────────────┘
                              │
                              ▼          S7
   No                    ╱──────────────╲
  ◀─────────────────────  TIME PERIOD T
                           ELAPSED?
                          ╲──────────────╱
                              │ Yes       S8
                     ┌─────────────────┐
                     │ DETECT SUBSTANCE│
                     └─────────────────┘
                              │
                              ▼
                     ┌─────────────────┐
                     │       END       │
                     └─────────────────┘
```

FIG.4

# FIG.5

# FIG.6

```
          ┌─────────────────┐
          │    SUBSTANCE    │
          │    DETECTION    │
          │   PROCESSING    │
          └─────────────────┘
                  │
     ┌────────────┼───────────
     │            ▼
     │    ┌─────────────────┐  ⌐S1
     │    │ DETECT DISTANCE L│
     │    └─────────────────┘
     │            │
     │            ▼
     │          ╱────╲        ⌐S2
  No │    ◄── L1≦L≦L2 ?
     │          ╲────╱
     │            │
     │           Yes          ⌐S6
     │            ▼
     │    ┌─────────────────┐
     │    │   TAKE IN GAS   │
     │    └─────────────────┘
     │            │
     │            ▼
     │          ╱────╲        ⌐S7
  No │    ◄── TIME PERIOD T
     │        ELAPSED?
     │          ╲────╱
     │            │
                 Yes          ⌐S8
                  ▼
          ┌─────────────────┐
          │ DETECT SUBSTANCE│
          └─────────────────┘
                  │
                  ▼
          ┌─────────────────┐
          │       END       │
          └─────────────────┘
```

## FIG.7

L2
(L2A)

L1
(L1A)

1C

20

25

RANGE
STORAGE

A

D ← DISTANCE
SENSOR

L

DETERMINER 21

2

3

10a

SUBSTANCE
SENSOR

CONTROLLER 24

11

L2
(L2B)

L1
(L1B)

10

B

L

DETECTION
RESULT

2

L2
(L2C)

L1
(L1C)

C

L

2

FIG.8

| GENERATION SOURCE | DISTANCE L1 | DISTANCE L2 |
|---|---|---|
| GENERATION SOURCE A | L1A | L2A |
| GENERATION SOURCE B | L1B | L2B |
| GENERATION SOURCE C | L1C | L2C |
| ⋮ | ⋮ | ⋮ |

## FIG.9

## FIG.10

| DETECTION RESULT | DISTANCE L[mm] |
|:---:|:---:|
| 10 | 21 |
| 20 | 25 |
| 15 | 18 |
| ⋮ | ⋮ |

# FIG.11

# FIG.12

CORRECTION VALUE
dV

g(L)

DISTANCE
L

O

# FIG.13

FIG.14

# FIG.15

# FIG.16

# FIG.17A

# FIG.17B

FIG.17C

FIG.17D

## INTERNATIONAL SEARCH REPORT

| | International application No. |
|---|---|
| | **PCT/JP2022/029810** |

### A.    CLASSIFICATION OF SUBJECT MATTER

**G01N 5/02**(2006.01)i; **G01N 27/00**(2006.01)i
FI:   G01N5/02 A; G01N27/00 K

According to International Patent Classification (IPC) or to both national classification and IPC

### B.    FIELDS SEARCHED

Minimum documentation searched (classification system followed by classification symbols)

G01N5/02; G01N27/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C.    DOCUMENTS CONSIDERED TO BE RELEVANT

| Category* | Citation of document, with indication, where appropriate, of the relevant passages | Relevant to claim No. |
|---|---|---|
| X | JP 2008-525771 A (INFICON GMBH) 17 July 2008 (2008-07-17)<br>    paragraphs [0007], [0014]-[0023] | 1-4-5 |
| Y | | 6-7 |
| X | US 4749553 A (LOPEZ, Benjamin L.) 07 June 1988 (1988-06-07)<br>    column 3, line 30 to column 4, line 20, column 9, line 37 to column 10, line 63 | 8-10 |
| Y | | 11-15 |
| Y | WO 2020/003532 A1 (NEC CORPORATION) 02 January 2020 (2020-01-02)<br>    paragraphs [0028], [0052] | 6 |
| Y | JP 2020-012732 A (FUJITSU LTD) 23 January 2020 (2020-01-23)<br>    paragraphs [0070]-[0167] | 7 |
| Y | JP 2010-102374 A (RIKEN KEIKI CO LTD) 06 May 2010 (2010-05-06)<br>    paragraphs [0030]-[0033] | 11-15 |
| A | JP 07-113774 A (TOYOTA MOTOR CORP) 02 May 1995 (1995-05-02)<br>    paragraphs [0018]-[0021] | 1-15 |

☑ Further documents are listed in the continuation of Box C.      ☑ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **21 September 2022** | **04 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

<table>
<tr><td colspan="2"></td><td>International application No.<br><br>**PCT/JP2022/029810**</td></tr>
</table>

**C.     DOCUMENTS CONSIDERED TO BE RELEVANT**

| Category* | Citation of document, with indication, where appropriate, of the relevant passages | Relevant to claim No. |
|---|---|---|
| A | JP 6765043 B1 (MEDICAL NET INC) 07 October 2020 (2020-10-07)<br>paragraph [0028] | 1-15 |

Form PCT/ISA/210 (second sheet) (January 2015)

**INTERNATIONAL SEARCH REPORT**
Information on patent family members

International application No.

**PCT/JP2022/029810**

| Patent document cited in search report | | | Publication date (day/month/year) | Patent family member(s) | | | Publication date (day/month/year) |
|---|---|---|---|---|---|---|---|
| JP | 2008-525771 | A | 17 July 2008 | US 2008/0000288 A1 paragraphs [0007], [0015]-[0025] WO 2006/069877 A1 EP 1828738 A1 DE 102004062102 A1 CN 101084424 A | | | |
| US | 4749553 | A | 07 June 1988 | WO 1988/008085 A1 AU 1547188 A | | | |
| WO | 2020/003532 | A1 | 02 January 2020 | US 2021/0255156 A1 paragraphs [0042], [0068] | | | |
| JP | 2020-012732 | A | 23 January 2020 | (Family: none) | | | |
| JP | 2010-102374 | A | 06 May 2010 | (Family: none) | | | |
| JP | 07-113774 | A | 02 May 1995 | US 5531225 A column 8, line 44 to column 9, line 6 | | | |
| JP | 6765043 | B1 | 07 October 2020 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009204584 A

- JP 2021131733 A